(19) Europäisches Patentamt
European Patent Office
Office européen des brevets

(11) Publication number: 0 104 826
A2

# (12) EUROPEAN PATENT APPLICATION

(21) Application number: 83305314.3

(22) Date of filing: 12.09.83

(51) Int. Cl.³: A 61 K 31/435
//C07D471/04, (C07D471/04, 221/00, 209/00)

(30) Priority: 17.09.82 GB 8226500

(43) Date of publication of application: 04.04.84 Bulletin 84/14

(84) Designated Contracting States: BE CH DE FR GB IT LI LU NL SE

(71) Applicant: Fujisawa Pharmaceutical Co., Ltd.
3, Doshomachi 4-chome Higashi-ku
Osaka-shi, Osaka 541(JP)

(72) Inventor: Motohiro, Hino
No. 2-2-10 Midorigaoka
Ikeda-shi(JP)

(72) Inventor: Kunio, Nakahara
No. 3-19-11, Namiki Sakuramura
Niihari-gun Ibaraki-ken(JP)

(72) Inventor: Hiroshi, Terano
No. 1-6-31, Tamaicho
Toyonaka-shi(JP)

(72) Inventor: Junji, Hosoda
No. 1-1-7, Chiyodabashi Chikusa-ku
Nygoya-shi(JP)

(72) Inventor: Masanobu, Kohsaka
No. 5-26-8, Akasakadai
Sakai-shi(JP)

(72) Inventor: Hatsuo, Aoki
No. 4-13-3, Hata
Ikeda-shi(JP)

(72) Inventor: Hiroshi, Imanaka
No. 4-19-25, Sakura Shimamotocho
Mishima-gun Osaka-fu(JP)

(74) Representative: Shipley, Warwick Grenville Michael
VENNER, SHIPLEY & CO. 368 City Road
London EC1V 2QA(GB)

## (54) Immunomodulator and method of use for immunomodulation.

(57) The present invention provides a pharmaceutical composition comprising, as active ingredient, swainsonine or a pharmaceutically acceptable salt thereof.

The present invention also provides a method of use of swainsonine or of a pharmaceutically acceptable salt thereof for the therapeutic treatment of diseases caused by or accompanied by depression of an immunoactivity.

EP 0 104 826 A2

0104826

# IMMUNOMODULATOR AND METHOD OF USE FOR IMMUNOMODULATION

This invention relates to an immunomodulator and a method of use for immunomodulation. Particularly, this invention relates to an immunomodulator comprising as an active ingredient swainsonine or its pharmaceutically acceptable salt and to a method of use of swainsonine or its pharmaceutically acceptable salt for immunomodulation.

Swainsonine to be used in this invention is the known substance possessing an α-mannosidase-inhibiting activity which was isolated from the plant Swainsona canescens and has the following chemical structure (c.f. Australian Journal of Chemistry, 1979, 32, P. 2257-2264).

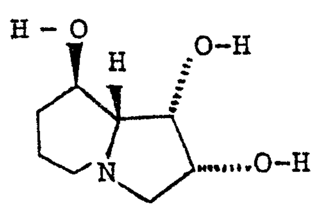

Any medical use of swainsonine, however, has not yet been known.

The inventors of this invention, as a result of an extensive study, have found that swainsonine and its pharmaceutically acceptable salt possess an immunomodulating activity such as restoration of a depressed-immunity, and accordingly are useful as an immunomodulator for therapeutic treatment of diseases caused by (or accompanied with) depression of an immuno-activity.

Preferred examples of the pharmaceutically acceptable salt of swainsonine may include an acid addition salt with an organic or an inorganic acid such as methane sulfonate, hydrochloride, sulfate, nitrate, phosphate or the like.

Swainsonine and its pharmaceutically acceptable salt are useful as an immunomodulator for therapeutic treatment of diseases of human-being and animal caused by (or accompanied with) depression of an immuno-activity, and further for therapeutic treatment of disease of immuno-insufficiency due to old age or the like.

As such diseases there may be exemplified tumor, infectious diseases, allergistic diseases, autoimmune diseases, steroid-dependent diseases and the like.

Swainsonine and its pharmaceutically acceptable salt are also useful for prevention of a subsidiary ill effect due to administration of medicines such as an antitumor agent, an antimicrobial agent, an antiinflammatory agent including a steroid hormone or the like. Namely, among these medicines, administration of them may cause occasionally, as a subsidiary ill effect, depression

of an immuno-activity, and swainsonine and its pharmaceutically acceptable salt prevent such a subsidiary ill effect, and promote the therapeutic effect of these medicines.

As examples of such medicines, there may be exemplified cyclophosphamide, cortisone acetate, vinblastine, vincristine, adriamycin, 6-mercaptopurine, 5-fluorouracil, mitomycin C, chloramphenicol and the like.

Accordingly, swainsonine and its pharmaceutically acceptable salt can be used as an immunomodulator for therapeutic treatment of diseases caused by (or accompanied with) depression of an immunoactivity alone or together with medicines such as an antitumor agent, an antimicrobial agent, an antiinflammatory agent or the like.

For the purpose of showing utility of swainsonine for an immunomodulator, pharmacological test data of swainsonine are illustrated in the followings.

Test 1 : <u>Competitive effect of swainsonine against immunosuppressive factor obtained from tumor bearing mice serum</u> :

Method :

1) Preparation of immunosuppressive factor from tumor bearing mice serum.

Mice. Female ICR/JCL (8 weeks of age) mice were obtained from Seidokyo, Shizuoka, Japan.

Tumor. Sarcoma 180 (S-180) cells maintained in vivo in ICR mouse in ascites form, were used for this experiment.

Preparation of immunosuppressive factor.

ICR mice were intraperitonealy inoculated with 0.2 ml of S-180 suspension ($5x10^6$ cells/ml). S-180 bearing mice were bled from heart under light ether anesthesia with sterile synringe between 7 and 9 days after transplantation and serum was collected.

Immunosuppressive factor was partially purified from the S-180 tumor bearing mice serum according to the method described by SE-KYUNG OH and F.L.MOOLTEN (J. Immunol., 127, 2300-2307, 1981). Briefly, the serum (50 ml) was delipidated with 1/10 vol. of 4% phosphotungstic acid and 1/40 vol of 2M magnesium chloride and centrifuged at 6000 x G for 10 min. Excess phosphotungstic acid and magnesium ion were removed by dialysis against phosphate-buffered saline (PBS: 0.15 M sodium chloride and 0.01 M phosphate buffer, pH 7.4). Then, partially delipidated serum was precipitated with ammonium sulfate at 50% saturation. The supernatant was removed by centrifugation at 20000 x G for 30 min. The precipitates were redissolved in a small volume of water and dialysed against PBS overnight at 4°C. The dialysed solution was chromatographed on Sephadex G-200 column in PBS. Each eluate (15 ml) was assayed for suppressive activity.

2) Assay method of immunosuppressive activity

Immunosuppressive activity was measured by assay for inhibition of the mitogen-induced mouse spleen cell proliferation.

3) Suppression of mitogen-induced mouse spleen cell

proliferation and its restoration by swainsonine in vitro.

Mitogenic activities for mouse spleen cells in vitro :

a) Mice. Female BALB/C strain (8 weeks of age) was used.
b) Tissue culture medium : The tissue culture medium employed was a complete medium designated Roswell Park Memorial Institute(RPMI)-1640. All media employed contained 100 units/ml of penicillin G and 100 µg/ml of streptomycin sulfate and 5% fetal calf serum.
c) Spleen cell preparation : Spleens were removed under sterile conditions and washed with Hanks solution and then teased in the tissue culture medium. The cells were suspended in the tissue culture medium to contain 5 x $10^5$ cells/ml.
d) Culture conditions : Into each hole of Microtiteration plate (Falcon No. 3040) were poured 0.1 ml of the above cells suspension and 0.1 ml of the prescribed concentrate of the compound and/or immunosuppressive factor described above. Concanavalin A (con A) was utilized for cell stimulation at a final concentration of 1 µg/ml. The culture was incubated in triplicate at 37°C in a humidified atmosphere (95% air, 5% $CO_2$) for 48 hours.
e) Assay for mitogen induced mouse spleen cells proliferation. The mitogen induced mouse spleen cells proliferation was assayed for tritiated thymidine ($^3$H-thymidine) incorporation. In all tests, 20 µl of 10 micro curie (µCi)/ml of $^3$H-thymidine was added to each hole for the 48 hours of culture. After further 24 hours incubation, the culture was terminated.

The resultant cells were filtered with filter paper, Whatman GF83 and washed successively with saline and with 5% trichloroacetic acid. The filter paper was dried and placed in a scintillator(toluene 1 liter containing 0.1 g of p-bis(5-phenyl oxazole)-benzene and 4 g of 2,5-diphenyloxazole), and $^3$H-thymidine incorporated into DNA was measured.

The suppressive effect of immunosuppressive factor on mitogenic response of spleen cells and its restoration by swainsonine were shown in Table 1. Immunosuppressive factor from the tumor bearing mice serum profoundly suppressed the mitogen induced stimulation of $^3$H-thymidine incorporation by mouse spleen cell (Table 1). This suppression was dose-dependent. The addition of swainsonine to the culture contained immunosuppressive factor, prevented the suppression. This result indicates that swainsonine has the capacity to restore the depression of mitogenic responses of mouse spleen cell by immunosuppressive factor. Furthermore, the addition of swainsonine to mouse spleen cell culture did not affected mitogenic activity of mouse spleen cell or spleen cell viability. When Con A and swainsonine were added simultaneously to culture, Con A induced stimulation of $^3$H-thymidine incorporation into mouse spleen cell was increased 10 times as much as that seen with Con A only.

This stimulation of mitogenecity of Con A on spleen cells by swainsonine indicates that the substance is capable of enhancing the mitogenic

responses of suppressor T-cells and may be used as an immunosuppressant in human being with allergistic diseases, autoimmune diseases and the like.

Table 1.

| Experiment No. | Treatment of spleen cell added | $^3$H-thymidine uptake (cpm)* |
|---|---|---|
| 1 | Non treated control | 1,616 |
| 2 | Concanavalin A | |
| | 3 μg/well | 121,245 |
| | 1 | 61,975 |
| | 0.3 | 13,146 |
| 3 | Immunosuppressive factor | |
| | 8 μl/well | 320 |
| | 6 | 302 |
| | 4 | 420 |
| | 2 | 1,310 |
| 4 | Swainsonine | |
| | 20 μg/well | 1,260 |
| | 2 | 1,726 |
| | 0.2 | 1,734 |
| | 0.02 | 1,712 |
| | 0.002 | 1,598 |
| | 0.0002 | 1,311 |
| 5 | Concanavalin A 1 μg/well + Immunosuppressive factor | |
| | 8 μl/well | 1,617 |
| | 6 | 2,626 |
| | 4 | 21,589 |
| | 2 | 60,308 |
| 6 | Concanavalin A 0.3 μg/well + swainsonine | |
| | 20 μg/well | 68,778 |
| | 2 | 100,480 |
| | 0.2 | 118,071 |
| | 0.02 | 108,748 |
| | 0.002 | 105,850 |
| | 0.0002 | 67,017 |
| 7 | Concanavalin A 1 μg/well + Immunosuppressive factor 6 μl/well + swainsonine | |
| | 20 μg/well | 37,746 |
| | 2 | 53,500 |
| | 0.2 | 70,890 |
| | 0.02 | 66,858 |
| | 0.002 | 69,313 |

* Note) The cpm is the mean of triplicate cultures.

Test 2 : Immunosuppressive effect of tumor bearing mouse serum on mixed lymphocyte culture and its restoration by swainsonine

Female C57BL/6 mice, 8 weeks in age, and female BALB/c mice, 8 weeks in age were sacrificed, spleens were aseptically removed and single cell suspensions were prepared as sources of stimulating and responding cells, respectively. Prior to culture, the stimulator cells were treated with 200 µg/ml of mitomycin C for 45 minutes at 37°C, followed by three washes with PBS.

They were established at a final concentration of 0.2 x $10^6$ cells/0.2 ml of reaction mixture and cultured in microtiteration plate (Falcon, No.3040) using RPMI medium supplemented with 10% fetal bovine serum and 5 x $10^{-5}$M 2-mercaptoethanol. The culture was continued for 120 hours and pulse-labeled with $^3$H-thymidine for 24 hours before the termination of incubation. The cells were collected and radioactivity was counted.

Immunosuppressive factor from tumor bearing mouse serum inhibited $^3$H-thymidine uptake into mixed lymphocyte culture (MLC)-stimulated spleen cells.

When the immunosuppressive factor was incubated with swainsonine, the inhibitory activity was removed.

The addition of swainsonine to MLC enhanced remarkably the MLC-stimulated spleen cells $^3$H-thymidine uptake.

This result indicates that swainsonine is capable of inducing killer T-cell generation.

Table 2.

| Experiment No. | Reaction | $^3$H-thymidine uptake (cpm)* |
|---|---|---|
| 1 | Unstimulated spleen cell (responder alone) | 4,401 |
| 2 | MLC without immunosuppressive factor | 18,866 |
| 3 | MLC with immunosuppressive factor | |
| | 3 µl | 3,278 |
| | 5 µl | 2,331 |
| 4 | MLC with immunosuppressive factor (3 µl) + swainsonine | |
| | 2 µg/well | 23,928 |
| | 0.2 | 27,942 |
| | 0.02 | 31,595 |
| | 0.002 | 24,951 |
| | 0.0002 | 13,015 |
| | 0.00002 | 10,449 |
| 5 | MLC with immunosuppressive factor (5 µl) + swainsonine | |
| | 2 µg/well | 6,709 |
| | 0.2 | 7,928 |
| | 0.02 | 7,679 |
| | 0.002 | 10,918 |
| | 0.0002 | 6,377 |
| | 0.00002 | 3,737 |
| 6 | MLC with swainsonine | |
| | 2 µg/well | 33,104 |
| | 0.2 | 38,077 |
| | 0.02 | 33,686 |
| | 0.002 | 29,688 |
| | 0.0002 | 21,823 |
| | 0.00002 | 20,653 |

* Note) The cpm is the mean of triplicate cultures.

Test 3 : Immunosuppressive effect of tumor bearing mouse serum on antibody forming capacities and its restoration by swainsonine :

Splenic lymphocytes producing antibody against sheep blood red cells (SRBC) were quantitated using Cunningham modification (Cunningham, A.J., Nature, 207, 1106-1107, 1965) of the Jerne plaque assay.
Female $BDF_1$ (8 weeks in age) mice were injected intravenously with 0.2 ml of immunosuppressive factor from 4 days before immunization with 5 x $10^8$ SRBC, every day for 4 days. Samples were administered to mice intraperitoneally four times before immunization. Mice were sacrificed 4 days after the injection of SRBC, and the number of direct plaque-forming cells per spleen was determined.

Table 3 shows immunosuppressive activity of suppressive factor against antibody forming capacities in vivo and its restoration by swainsonine.
Immunosuppressive factor from the tumor bearing mouse serum suppressed the antibody forming capacities against SRBC in mice.
The intraperitoneal administration of swainsonine restored the capacities of the mice injected immunosuppressive factor to produce antibody against SRBC. Swainsonine did not enhance the capacities of normal mice to produce antibody against SRBC.

Table 3.

| Substance | PFC-SRBC/spleen (average number of 5 mice/group) |
|---|---|
| Control (saline) | $19.25 \times 10^4$ |
| Immunosuppressive factor (0.3 ml/mouse/day) | $9.32 \times 10^4$ |
| Swainsonine (100 mg/kg/day) | $16.49 \times 10^4$ |
| Immunosuppressive factor (0.3 ml/mouse/day) + Swainsonine 100 mg/kg | $17.39 \times 10^4$ |
| 33 | $20.84 \times 10^4$ |
| 11 | $20.08 \times 10^4$ |
| 3.7 | $20.77 \times 10^4$ |

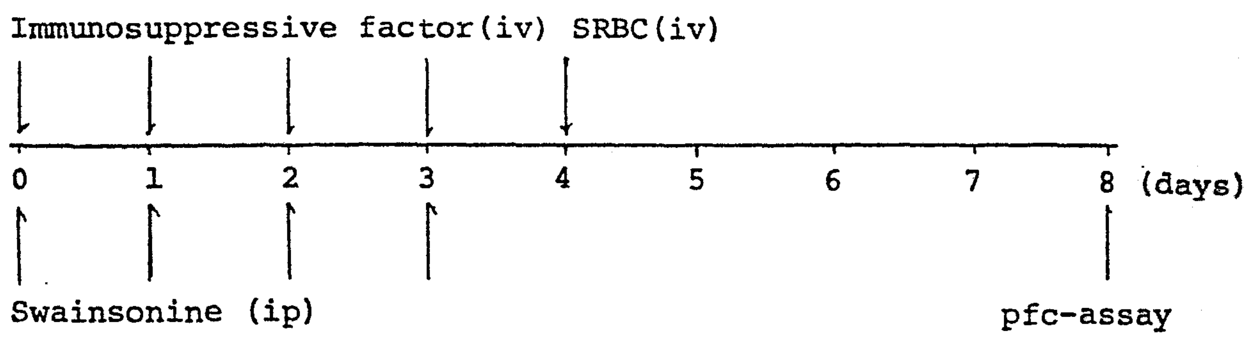


Test 4 : Depression of antibody forming capacities in tumor bearing mice and its restoration by the swainsonine :

Female ICR(8 weeks in age) mice were intraperitoneally inoculated with 0.2 ml of Sarcoma 180 suspension ($5 \times 10^6$ cells/ml) at day 0. Samples were administered to mice intraperitoneally from 7 days before immunization with $5 \times 10^8$ SRBC, every day for five days.

The number of direct plaque forming cells per spleen was measured five days after immunization.

Table 4 shows the immunosuppressive effect on the development of tumor and its restoration of immune response by swainsonine in tumor bearing mice. The administration of swainsonine restored the capacities of the tumor bearing mice to produce antibody against SRBC.

Table 4.

| | | PFC-SRBC/spleen (average number of 5 mice/group) |
|---|---|---|
| Control (saline) | | $139.1 \times 10^4$ |
| Tumor bearer | | $46.4 \times 10^4$ |
| Swainsonine 100 mg/kg | | $118.7 \times 10^4$ |
| Tumor bearer | Swainsonine 100 mg/kg | $126.7 \times 10^4$ |
| | 33 | $123.5 \times 10^4$ |
| | 11 | $71.6 \times 10^4$ |
| | 3.7 | $61.6 \times 10^4$ |

Schedule

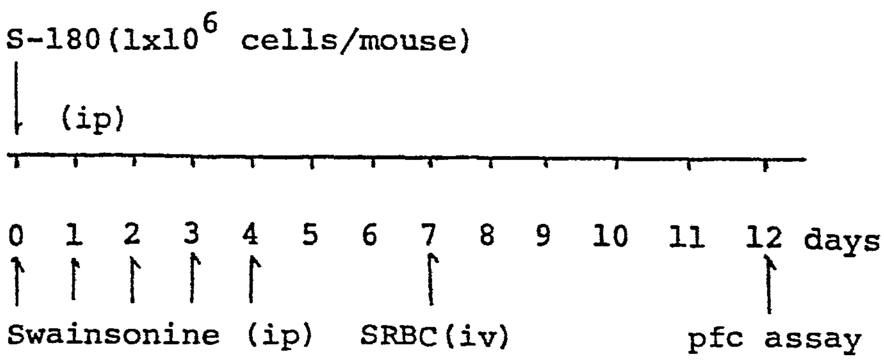

Test 5 : Immunosuppressive effect of mitomycin C on antibody forming capacities and its restoration by swainsonine :

Female $BDF_1$ (8 weeks in age) mice were injected intraperitoneally with mitomycin C (1 mg/kg) from four days before immuization with 5 x $10^8$ SRBC, every day for three days. Samples also were administered to mice intraperitoneally from four days before immunization, every day for five days.

At day 9, mice were sacrificed and the number of direct plaque forming cells per spleen was measured by using Cunningham modification method.

Table 5 shows immunosuppressive activity of mitomycin C in vivo and its restoration by swainsonine. The administration of swainsonine restored the capacities of the mice injected mitomycin C to produce antibody against SRBC.

Table 5.

| Substance | | PFC-SRBC/spleen (average number of 5 mice/group) |
|---|---|---|
| Control (saline) | | $19.73 \times 10^4$ |
| Swainsonine 100 mg/kg | | $21.66 \times 10^4$ |
| Mitomycin C (1.0 mg/kg) | Saline | $12.65 \times 10^4$ |
| | Swainsonine 100 mg/kg | $21.79 \times 10^4$ |
| | 33 | $20.90 \times 10^4$ |
| | 11 | $20.90 \times 10^4$ |
| | 3.7 | $21.31 \times 10^4$ |

Schedule

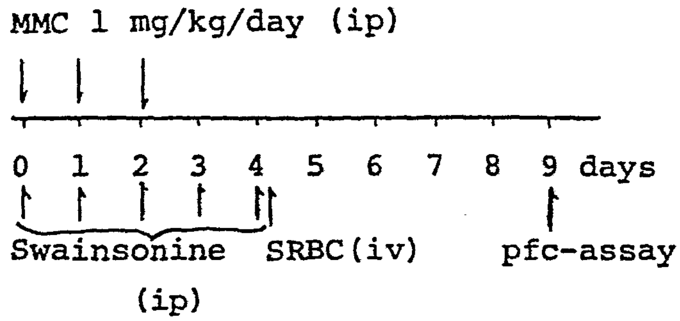


Test 6 : <u>Immunosuppressive effect of cyclophosphamide on antibody forming capacities and its restoration by swainsonine</u> :

Female $BDF_1$ (8 weeks in age) mice were injected intraperitoneally with cyclophosphamide (22.5 mg/kg) 4 days before immunization with 5 x $10^8$ SRBC. Samples were administered to mice i.p. from four days before immunization, every day for five days.

Mouse splenic lymphocytes producing antibody against SRBC were quantitated by using Cunningham modification method described previously.

Table 6 shows immunosuppressive activity of cyclophosphamide in vivo and its restoration by swainsonine. The administration of swainsonine restored the capacities of the mice injected cyclophosphamide to produce antibody against SRBC.

Schedule

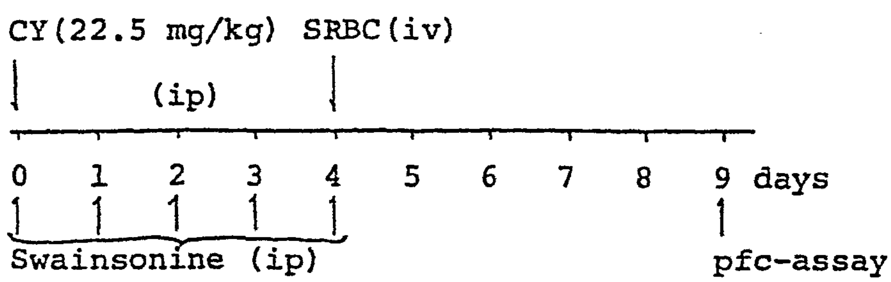

Table 6.

| Substance | | PFC-SRBC/spleen (average number of 5 mice/group) |
|---|---|---|
| Control (saline) | | $16.94 \times 10^4$ |
| Swainsonine (100 mg/kg) | | $16.54 \times 10^4$ |
| Cyclophosphamide (22.5 mg/kg) | Saline | $8.46 \times 10^4$ |
| | Swainsonine 100 mg/kg | $16.48 \times 10^4$ |
| | 33 | $17.19 \times 10^4$ |
| | 11 | $16.52 \times 10^4$ |
| | 3 | $21.53 \times 10^4$ |

Test 7 : <u>Prevention from dexamethasone-induced thymus atrophy by swainsonine</u> :

Five week-old ddY mice(male) were used. Samples were given subcutaneously twice per day from day 0 to day 2 of the experiment. Five hours after first medication on day 0, mice were supplied with drinking water containing 1 µg/ml of dexamethasone.
At day 2, the weight of thymus of the treated mice was compared with that of the nontreated controls. The result is shown in Table 7.
Swainsonine prevents the mouse from atrophy of thymus induced by dexamethasone in mice. The fact indicates that the compound is capable of restoring the immunosuppression induced by dexamethasone.

Table 7.

| Sample | Dexamethasone treatment | Thymus weight[1)] |
|---|---|---|
| - | No | 76.8 |
| - | Yes | 57.2 |
| 50 mg/kg | Yes | 72.4 |
| 15 | Yes | 78.8 |
| 5 | Yes | 73.0 |

1) Thymus weight (mg/mouse) (n=5)

Test 8 : Effect of swainsonine on murine transplantable tumors :

Sarcoma-180 (S-180) cells (1 x $10^6$ cells) were inoculated to ICR (female, 8 weeks old) mice intraperitoneally and then after, swainsonine was administered intraperitoneally daily for 5 days. The total tumor cell packed volume in ascites was measured 15 days after tumor cell inoculation. As shown in Table 8 Swainsonineinhibits growth of transplantable tumors in mice.

Table 8.

| Dose (mg/kg/day) | Total cell packed volume (ml)[1)] |
|---|---|
| 100 | 0, 0, 0, 0, 0 |
| 30 | 0, 0, 0, 0, 0 |
| 10 | 5, 12, 23, 33, 38 |
| 3 | 22, 32, 40, 40, 47 |
| Control | 35, 38, 39, 45, 48 |

1) 1000 rpm for 10 minutes

Acute toxicity of swainsonine in ddY mice is above 1g/kg by intravenous injection.

The pharmaceutical composition of this invention can be used in the form of a pharmaceutical preparation, for example, in solid, semisolid or liquid form, which contains an active substance of this invention in admixture with an organic or inorganic carrier or excipient suitable for external, enteral or parenteral applications. The active ingredient may be compounded, for example, with the usual non-toxic, pharmaceutically acceptable carriers for tablets, pellets, capsules, suppositories, solutions, emulsions, suspensions, and any other form suitable for use. The carriers which can be used are water, glucose, lactose, gum acacia, gelatin, mannitol, starch paste, magnesium trisilicate, talc, corn starch, keratin, colloidal silica, potato starch, urea and other carriers suitable for use in manufacturing preparations, in solid, semisolid, or liquid form, and in addition auxiliary, stabilizing, thickening and coloring agents and perfumes may be used. The pharmaceutical compositions can also contain preservative or bacteriostatic agents to keep the active ingredient in the desired preparations stable in activity. The active object compound is included in the pharmaceutical composition in an amount sufficent to produce the desired therapeutic effect upon the process or condition of diseases.

For applying this composition to humans, it is preferably to apply it by intravenous, intramuscular or oral administration. While the dosage or therapeutically effective amount of the object compound of this invention varies from and also depends upon the age and condition of each individual patient to be treated, a daily dose of about 0.1-100 mg of the active

ingredient/kg of a human being or an animal is generally give for treating diseases, and an average single dose of about 50 mg, 100 mg, 250 mg, and 500 mg is generally administered.

In case of use of swainsonine or its pharmaceutically acceptable salt and an antitumor, antibacterial, or antiinflammatory agent, the ratio of swainsonine or its pharmaceutically acceptable salt and an antitumor, antibacterial or antiinflammatory agent may vary with the kinds of disease and said agent, but may usually be in a range of 1:0.001 to 1:10 by weight.

The following Examples are given for the purpose of illustrating this invention.

Example 1

Swainsonine (250 mg) was dissolved in a sterile distilled water (2 ml) and adjusted to pH 7.0 - 7.2 with 1N hydrochloric acid. The resultant solution was distributed into vials and then lyophilized.
Whenever the vial is required for use, 2 ml of sterile distilled water for injection is added to the vial and then the aqueous solution is administered by injection.

Example 2

| | |
|---|---|
| Swainsonine | 250 mg |
| 100 Meshed lactose | 50 mg |
| Starch | 50 mg |
| Magnesium stearate | 3 mg |

The above ingredients were mixed and then inserted into a hard gelatin capsule in a conventional manner.

Example 3

A suitable formulation for a tablet consists of the following mixture.

| | |
|---|---|
| Swainsonine | 250 mg |
| Lactose | 60 mg |
| Hydroxypropyl cellulose | 6 mg |
| Starch | 30 mg |
| Magnesium stearate | 3 mg |

We claim

1. A pharmaceutical composition comprising as an active ingredient swainsonine or its pharmaceutically acceptable salt.

2. A method of use of swainsonine or its pharmaceutically acceptable salt for therapeutic treatment of diseases caused by (or accompanied with) depression of an immuno-activity.

3. An immunomodulating agent comprising as an active ingredient swainsonine or its pharmaceutically acceptable salt.

4. An immunomodulating agent comprising as an active ingredient swainsonine or its pharmaceutically acceptable salt and an antitumor, antibacterial or antiinflamatory agent.

5. A method of use of swainsonine or its pharmaceutically acceptable salt and an antitumor, antibacterial or antiinflamatory agent for preventing depression of an immuno-activity, caused by administration of said agent.

6. A method of claim 2, wherein the disease accompained with depression of an immuno-activity is tumor, infections diseases, allergistic diseases, autoimmune diseases or steroid-dependent diseases.

7. A method of use of swainsonine or its pharmaceutically acceptable salt and an antitumor agent for preventing depression of an immuno-activity, caused by

administration of said antitumor agent.

8. A method of claim 7, wherein the antitumor agent is mitomycin C or cyclophosphamide.